# EUROPEAN PATENT SPECIFICATION

(11) **EP 1 439 848 B1**
(45) Date of publication and mention of the grant of the patent: **26.11.2014**
(21) Application number: 02766203.0
(22) Date of filing: 29.08.2002
(51) Int. Cl.: A61K 38/00

(54) **L-METHIONINE AS A STABILIZER FOR NESP/EPO IN HSA-FREE FORMULATIONS**
L-METHIONIN ALS STABILISATOR FÜR NESP/EPO IN HSA-FREIEN FORMULIERUNGEN
L-METHIONINE UTILISEE COMME STABILISATEUR DE NESP/EPO DANS DES FORMULATIONS EXEMPTES DE HSA

(30) Priority: 30.08.2001 US 945517
(43) Date of publication of application: 28.07.2004
(73) Proprietor: Kirin-Amgen, Inc., Wilmington, DE 19801 (US)
(72) Inventor: LI, Tiansheng, Newbury Park, CA 91320 (US); CHANG, Byeong, S., Thousand Oaks, CA 91320 (US); SLOEY, Christopher, Sherman Oaks, CA 91411 (US)
(74) Representative: Bassil, Nicholas Charles
(86) International application number: PCT/US2002/027855
(87) International publication number: WO 2003/020299

(56) References cited:
- CA-A1- 2 381 229
- US-A- 4 835 260
- US-A- 5 272 135
- US-A- 5 929 028
- US-A1- 2002 037 841
- ELLIOTT S G ET AL: "RATIONAL DESIGN OF NOVEL ERYTHROPOIESIS STIMULATING PROTEIN (ARANESPTM): A SUPER-SIALATED MOLECULE WITH INCREASED BIOLOGICAL ACTIVITY" BLOOD, AMERICAN SOCIETY OF HEMATOLOGY, US, vol. 96, no. 11 PART 1, 16 November 2000 (2000-11-16), pages 82A-83A, XP009082985 ISSN: 0006-4971

## Description

### BACKGROUND OF THE INVENTION

Due to recent advances in genetic and cell engineering technologies, proteins known to exhibit various pharmacological actions *in vivo* are capable of being produced in large amounts for pharmaceutical applications. Such proteins include erythropoietin (EPO), granulocyte colony-stimulating factor (G-CSF), interferons (alpha, beta, gamma, consensus), tumor necrosis factor binding proteins (TNFbp), interleukin-1 receptor antagonist (IL-1ra), brain-derived neurotrophic factor (BDNF), keratinocyte growth factor (KGF), stem cell factor (SCF), megakaryocyte growth differentiation factor (MGDF), osteoprotegerin (OPG), glial cell line derived neurotrophic factor (GDNF), obesity protein (OB protein), and novel erythropoiesis stimulating protein (NESP).

EPO is a glycoprotein hormone necessary for the maturation of erythroid progenitor cells into erythrocytes. It is produced in the kidney and is essential in regulating levels of red blood cells in the circulation. Conditions marked by low levels of tissue oxygen signal increased production of EPO, which in turn stimulates erythropoiesis. A loss of kidney function as is seen in chronic renal failure (CRF), for example, typically results in decreased production of EPO and a concomitant reduction in red blood cells. Human urinary EPO was purified by Miyake et al., J. Biol. Chem., 252:5558 (1977) from patients with aplastic anemia. However, the amount of purified EPO protein obtained from this source was insufficient for therapeutic applications. The identification and cloning of the gene encoding human EPO and expression of recombinant protein was disclosed in U.S. Patent No. 4,703,008 to Lin.

A method for purification of recombinant human erythropoietin from cell medium is disclosed in U.S. Patent No. 4,667,016 to Lai et. al.

The production of biologically active EPO from mammalian host cells has made available, for the first time, quantities of EPO suitable for therapeutic applications. In addition, knowledge of the gene sequence and the increased availability of purified protein has led to a better understanding of the mode of action of this protein.

Both human urinary derived EPO (Miyake et al. supra) and recombinant human EPO expressed in mammalian cells contain three N-linked and one O-linked oligosaccharide chains which together comprise about 40% of the total molecular weight of the glycoprotein. N-linked glycosylation occurs at asparagine residues located at positions 24, 38 and 83 while O-linked glycosylation occurs at a serine residue located at position 126 (see Lai et al., J. Biol. Chem., 261:3116 (1986); Broudy et al., Arch. Biochem. Biophys, 265:3219 (1988)). The oligosaccharide chains have been shown to be modified with terminal sialic acid residues with N-linked chains typically having up to four sialic acids per chain and O-linked chains having up to two sialic acids. An EPO polypeptide may therefore accommodate up to a total of 14 sialic acids.

Various studies have shown that alterations of EPO carbohydrate chains can affect biological activity. In one study, however, the removal of N-linked or O-linked oligosaccharide chains singly or together by mutagenesis of asparagine or serine residues that are glycosylation sites sharply reduces *in vitro* activity of the altered EPO that is produced in mammalian cells; Dube et. al., J. Biol. Chem., 263:17516 (1988). However, DeLorme et al., Biochemistry, 31:9871-9876 (1992) reported that removal of N-linked glycosylation sites in EPO reduced *in vivo* but not in *vitro* biological activity.

The relationship between the sialic acid content of EPO and *in vivo* biological activity was disclosed by determining the *in vivo* activity of isolated EPO isoforms. It was found that a stepwise increase in sialic acid content per EPO molecule gave a corresponding stepwise increase in *in vivo* biological activity as measured by the ability of equimolar concentrations of isolated EPO isoforms to raise the hematocrit of normal mice; Egrie et al., Glycoconjugate J., 10:263 (1993). Those EPO isoforms having higher sialic acid content also exhibited a longer serum half-life but decreased affinity for the EPO receptor, suggesting that serum half-life is an important determinant of *in vivo* biological activity.

In the U.S., EPO has been used in the treatment of chronic renal failure maintained on dialysis as well as pre-dialysis, and in the treatment anemia secondary to chemotherapy treatment in cancer and in anemia associated with zidovudine treatment of HIV infection. Worldwide, EPO has been used to treat anemia associated with prematurity, sickle cell anemia, rheumatoid arthritis, and bone marrow transplantation; Markham et al., Drugs, 49:232-254 (1995).

NESP is a hyperglycosylated erythropoietin analog having five changes in the amino acid sequence of rHuEPO which provide for two additional carbohydrate chains. More specifically, NESP contains two additional N-linked carbohydrate chains at amino acid residues 30 and 88 (numbering corresponding to the sequence of human EPO) (see PCT publication no. 95/05465).

NESP is biochemically distinct from EPO, having a longer serum half-life and higher *in vivo* biological activity; Egrie et al., ASH 97, Blood, 90:56a (1997). NESP has been shown to have ~3 fold increase in serum half-life in mice, rats, dogs and man; Id. In mice, the longer serum half-life and higher *in vivo* activity allow for less frequent dosing (once weekly or once every other week) compared to rHuEPO to obtain the same biological response; Id.

A pharmacokinetic study demonstrated that, consistent with the animal studies, NESP has a significantly longer serum half-life than rHuEPO in chronic renal failure patients, suggesting that a less frequent dosing schedule may also be employed in humans; MacDougall, et al., J American Society of Nephrology, 8:268A (1997). A less frequent dosing schedule would be more convenient to both physicians and patients, and would be particularly helpful to those patients involved in self-administration. Other advantages to less frequent dosing may include less drug being introduced into patients, a reduction in the nature or severity of the few side-effects seen with rHuEPO administration, and increased compliance.

Although commercially available EPO and NESP formulations are generally well tolerated and stable, consideration should be given to the fact that, under extreme conditions, such proteins may be unstable and undergo various undesirable physiochemical degradations during manufacturing, handling, and storage conditions. Such degradations include aggregation, inactivation, and oxidation of methionine residues, and such degradations may be accelerated by external factors such as heat and light, or in formulations that are free of human blood products such as albumin, or in multi-dose formulations which contain preservatives such as benzyl alcohol.

Methods of inhibiting oxidation in methionine-containing polypeptides have been described; Takruri et al., U.S. Patent No. 5,272,135 (December 21, 1993). Specifically, Takruri describes methods of inhibiting the oxidation of methionine residue(s) in liquid or semi-liquid preparations, said preparations comprising polypeptides having amino acid sequences comprising at least one methionine residue. The prevention of methionine oxidation is said to be accomplished by the addition of free L-methionine to the preparations in an amount sufficient to inhibit oxidation of the methionine residue(s) in the polypeptide. The oxidation of the methionine residues is said to be associated with the plastic containers, e.g., polypropylene or low density polyethylene (LDPE), which are readily permeable to oxygen, and within which the preparations are stored. The polypeptides contemplated for use in Takruri are growth factors, and the preparations tested are ophthalmic aqueous-based preparations of epidermal growth factor (EGF). Preparations containing EPO or NESP, or any other glycosylated protein are not discussed, nor are formulations which are HSA-free, multi-dose, or HSA-free multi-dose discussed.

CA 2,381,229 describes stabilised formulations containing proteins having a methionine residue such as G-CSF and the addition of methionine. US 5,929,028 describes a formulation containing gonadotrophin or gonadotrophin derivatives and methionine as a stabiliser.

### SUMMARY OF THE INVENTION

The present invention provides pharmaceutical formulations of a novel erythropoiesis stimulating protein (NESP) and further comprising methionine. Other embodiments of this aspect of the invention are as defined in the claims.

According to another aspect of the invention, there is provided a pharmaceutical formulation comprising a novel erythropoiesis stimulating protein, a preservative and methionine wherein said formulation demonstrates improved stability. Other embodiments of this aspect of the invention are as defined in the claims.

According to a further aspect of the invention, there is provided a method of stabilizing a pharmaceutical composition of a protein selected from erythropoietin (EPO) or novel erythropoiesis stimulating protein (NESP) against tryptophan, cysteine and/or histidine oxidation which comprises adding methionine to said composition in an amount sufficient to inhibit oxidation of an amino acid residue in the amino acid sequence of said EPO or NESP; wherein said amino acid residue is selected from the group consisting of tryptophan, cysteine, histidine and combinations thereof. Other embodiments of this aspect of the invention are as defined in the claims.

According to another aspect of the invention there is provided a pharmaceutical formulation comprising a protein selected from erythropoietin (EPO) or novel erythropoiesis stimulating protein (NESP) and methionine present in a concentration of about 0.5mM to 50mM, wherein said formulation demonstrates improves stability. Other embodiments of this aspect of the invention are as defined in the claims.

Said formulations also provide for a more stable formulation, even in extreme conditions wherein critical degradations induced by light, heat, impurities in additives, leacheates in the prefilled syringes, the manufacturing process, storage, transportation, and handling may otherwise occur.

Importantly, the formulations also demonstrate improved stability in HSA-free formulations and HSA-free multi-dose formulations containing preservatives, wherein the critical degradations may be more pronounced.

### BRIEF DESCRIPTION OF THE DRAWINGS

Figure 1 is a graph depicting the effect of free methionine on the aggregation of NESP during exposure to light. NESP in phosphate buffered saline was exposed to ultraviolet light for 4 hours at room temperature.
Figure 2 is a graph depicting the effect of free methionine on the aggregation of NESP in the presence of 1% benzyl alcohol during storage at 2-8°C. Samples containing 500 µg/mL of NESP were stored for 13 months.
Figure 3 is a graph depicting the effect of various additives and treatment on the oxidation of methionine 54 residue in NESP during incubation for 90 days at 37°C. % oxidation was determined by tryptic mapping followed by Reversed-phase HPLC and mass spectrometry.
Figure 4 is a graph depicting the effect of free methionine on the oxidation of NESP in a preserved formulation containing 1% benzyl alcohol. 0-20mM free methionine was tested and samples were incubated at 4°C for 56 days.
Figure 5 is a graph depicting the effect of free methionine on the oxidation of NESP in a preserved formulation containing 1% benzyl alcohol. 0-20mM free methionine was tested and samples were incubated at 29°C for 56 days.
Figure 6 compares the tryptic maps of EPO in solutions at pH 7.0 ± benzyl alcohol and ± free L-methionine.
Figure 7 is a graph comparing NESP methionine oxidation rates with and without purging (10 minutes) with nitrogen. % methionine oxidation is plotted versus benzaldehyde concentration. 0.1 mg/ml NESP was tested.
Figure 8 compares the tryptic maps of over-oxidized NESP samples. Met-54 was fully oxidized for all samples shown in the figure. Numbers depicted on the figure represent the concentration of methionine added to the samples.

### DETAILED DESCRIPTION OF THE INVENTION

"Excipient" is defined herein as a non-therapeutic agent added to a pharmaceutical composition to provide a desired effect, e.g. stabilization, isotonicity.

"Polypeptide" is defined herein as natural, synthetic, and recombinant proteins or peptides having more than about 10 amino acids, and having a desired biological activity.

As used herein, biologically active agents refers to recombinant or naturally occurring polypeptides, whether human or animal, useful for prophylactic, therapeutic or diagnostic application. The biologically active agent can be natural, synthetic, semi-synthetic or derivatives thereof. Contemplated active agents include peptides, small molecules, carbohydrates, nucleic acids, lipids, proteins, and analogs thereof. One skilled in the art will readily be able to adapt a desired biologically active agent to the compositions of present invention.

Proteins contemplated for use would include but are not limited to interferon consensus (see, U.S. Patent Nos. 5,372,808, 5,541,293 4,897,471, and 4, 695, 623),
granulocyte-colony stimulating factors (see, U.S. Patent Nos. 4,810,643, 4,999,291, 5,581,476, 5,582,823, and PCT Publication No. 94/17185),
interleukins (see, U.S. Patent No. 5,075,222),
erythropoietins (see, U.S. Patent Nos. 4,703,008, 5,441,868, 5,618,698 5,547,933, and 5,621,080),
stem cell factor (PCT Publication Nos. 91/05795, 92/17505 and 95/17206),
osteoprotegerin (PCT Publication No. 97/23614),
novel erythropoiesis stimulating protein (NESP) (PCT Publication No. 95/05465),
leptin (OB protein) (see PCT publication Nos. 96/40912, 96/05309, 97/25424, 97/26335 and 97/06816 megakaryocyte growth differentiation factor (see, PCT Publication No. 95/26746),
,tumor necrosis factor-binding protein (TNF-bp), interleukin-1 receptor antagonist (IL-1ra), brain derived neurotrophic factor (BDNF), glial derived neurotrophic factor (GDNF), keratinocyte growth factor (KGF) and thrombopoietin. The term proteins, as used herein, includes peptides, polypeptides, consensus molecules, analogs, derivatives or combinations thereof.

In general, EPO useful in the present invention has the sequence of human erythropoietin, or closely related analogues thereof. The EPO may be produced by mammalian cells outside the body, or it may be isolated from natural sources. Preferably, the EPO is recombinant human EPO (rHuEPO) produced as described in U. S. Patent No. 4,703,008 to Lin.

The amino acid sequence of EPO is that depicted herein in SEQ ID NO:1. The preferred host cells are Chinese Hamster Ovary (CHO) cells as described in Example 10 of the Lin patent. Other host cells known in the art, e.g. baby hamster kidney cells, may also be used to produce EPO useful in the present invention. While the procedures of Example 10 in the Lin patent are the preferred method for producing rEPO, modifications and changes could be made to that process as known in the art. The preferred concentration of EPO is 50 IU/mL - 500,000 IU/mL, and 750 IU/mL - 48,000 IU/mL is more preferred.

NESP of the present invention is a hyperglycosylated EPO analog comprising two additional glycosylation sites with an additional carbohydrate chain attached to each site. NESP was constructed using site-directed mutagenesis and expressed in mammalian host cells. Details of the production of NESP are provided in co-owned PCT publication no. 95/05465). New N-linked glycosylation sites for rHuEPO were introduced by alterations in the DNA sequence to encode the amino acids Asn-X-Ser/Thr in the polypeptide chain. DNA encoding NESP was transfected into Chinese Hamster Ovary (CHO) host cells and the expressed polypeptide was analyzed for the presence of additional carbohydrate chains. In a preferred embodiment, NESP will have two additional N-linked carbohydrate chains at residues 30 and 88. The numbering of the amino acid sequence is that of human erythropoietin (EPO). The amino acid sequence of NESP is that depicted herein in SEQ ID NO:2. It is understood that NESP will have the normal complement of N-linked and O-linked glycosylation sites in addition to the new sites. The preferred concentration of NESP is 1 µg/mL - 5000 µg/mL, and 10 µg/mL - 500 µg/mL is more preferred.

The EPO and NESP of the present invention may also include conservative amino acid changes at one or more residues in SEQ ID NOs:1 and 2. These changes do not result in addition of a carbohydrate chain and will have little effect on the biological activity of the analog. These are set forth in Table 1, below. See *generally,* Creighton, Proteins, passim (W.H. Freeman and Company, N.Y., 1984); Ford et al., Protein Expression and Purification 2:95-107 (1991).

**Table 1**

| Conservative Amino Acid Substitutions | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

Therapeutic uses of the compositions of the present invention depend on the biologically active agent used. One skilled in the art will readily be able to adapt a desired biologically active agent to the present invention for its intended therapeutic uses. Therapeutic uses for such agents are set forth in greater detail in the following publications hereby incorporated by reference including drawings. Therapeutic uses include but are not limited to uses for proteins like consensus interferon (see, U.S. Patent Nos. 5,372,808, 5,541,293),
, interleukins (see, U.S. Patent No. 5,075,222),
erythropoietins (see, U.S. Patent Nos. 4,703,008, 5,441,868, 5,618,698 5,547,933, 5,621,080, 5,756,349, and 5,955,422), granulocyte-colony stimulating factors (*see,* U.S. Patent Nos. 4,999,291, 5,581,476, 5,582,823, 4,810,643 and PCT Publication No. 94/17185,
megakaryocyte growth differentiation factor (see, PCT Publication No. 95/26746), stem cell factor (PCT Publication Nos. 91/05795, 92/17505 and 95/17206),
OB protein (see PCT publication Nos. 96/40912, 96/05309, 97/25424, 97/26335 and 97/06816),
and novel erythropoiesis stimulating protein (PCT Publication No. 95/05465).

In addition, the present compositions may also be used for manufacture of one or more medicaments for treatment or amelioration of the conditions the biologically active agent is intended to treat.

As relates specifically to NESP, the present invention provides for a method of raising and maintaining hematocrit in a mammal comprising administering a therapeutically effective amount of NESP in a pharmaceutical composition of the present invention, wherein the NESP is administered less frequently than an equivalent molar amount of rHuEPO to obtain a comparable target hematocrit. The dosing frequency of the present invention in order to reach a patient's optimal hematocrit range is less than three times per week. Dosing frequencies may be two times per week, one time per week, or less than one time per week, such as one time every other week, once per month or once every two months. The dosing frequency required to maintain a patient's target hematocrit is less than three times per week. Dosing frequencies may be two times per week, one time per week, or less than one time per week, such as one time every two weeks, once per month or once every two months.

The invention may be employed with any condition resulting in a decrease in red blood cell levels, such as anemia associated with a decline or loss of kidney function, (chronic renal failure) myelosuppressive therapy, cancer, viral infection, chronic disease and excessive loss of blood during surgical procedures.

It is envisioned that the formulations of the present invention will additionally contain a buffering agent, e.g., alkali salts (sodium or potassium phosphate or their hydrogen or dihydrogen salts), sodium citrate/citric acid, sodium acetate/acetic acid, and any other pharmaceutically acceptable ph buffering agent known in the art, to maintain the pH of the solution within a desired range. Mixtures of these buffering agents may also be used. The amount of buffering agent useful in the composition depends largely on the particular buffer used and the pH of the solution. For example, acetate is a more efficient buffer at pH 5 than pH 6 so less acetate may be used in a solution at pH 5 than at pH 6. The preferred pH of the preferred formulations will be in the range of 5.0 to 7.0, and pH-adjusting agents such as hydrochloric acid, citric acid, sodium hydroxide, or a salt thereof, may also be included in order to obtain the desired pH.

The formulations will also contain sorbitan mono-9-octadecenoate poly(oxy-1,2-ethanediyl) derivatives, including but not limited to, polysorbate 80 or polysorbate 20. Other derivatives are well known in the art. The amount of polysorbate 20 or 80 to be used will be in the range of 0.001% to 0.1% (w/v). The preferred amount is 0.005% (w/v) in the single use and multi-dose formulations.

In order to provide EPO and/or NESP pharmaceutical formulations having superior stability, free L-methionine will be included in the formulations. The amount of free L-methionine included will be in the range of 0.05mM to 50mM. In HSA-containing formulations, the preferred amount in the single use formulations is 0.05mM to 5mM, and the preferred amount in the multi-dose formulations is 1mM to 10mM. In HSA-free formulations, the preferred amount in the single use formulations is 0.05mM to 5mM, and the preferred amount in the multi-dose formulations is 1mM to 10mM.

Preservatives contemplated for use in the multi-dose formulations of the present invention include benzyl alcohol, benzalkonium chloride, chlorobutanol, cresol, phenol, and parabens. The amount of preservative included will be in the range of 0% to 2% (w/v) and the preferred amount in the formulations is 1% (w/v).

The formulations of the present invention may further include an isotonicity adjusting agent to render the solution isotonic and more compatible for injection. Typical tonicity agents are well known in the art and include but are not limited to sodium chloride, mannitol, glycine, and sorbitol. The preferred agent is sodium chloride within a concentration range of 0mM to 200mM.

It is also envisioned that other anti-oxidants may be included in the formulations of the present invention. Anti-oxidants contemplated for use in the preparation of the formulations include amino acids such as glycine and lysine, chelating agents such as EDTA and DTPA, and free-radical scavengers such as sorbitol and mannitol.

Preferred NESP formulations contemplated for use in the present invention will contain 1-5000 µg/mL NESP, 10mM to 30mM phosphate buffer, 100mM to 200mM NaCl, 0.001% to 0.1%(w/v) polysorbate 80, and 0.5mM to 50mM L-methionine, pH 5.0-7.0; and more preferably, 10-500 µg/mL NESP, 20mM phosphate buffer, 140mM NaCl, 0.005% (w/v) polysorbate 80, and 1mM L-methionine, pH 6.2.

Preferred EPO formulations contemplated for use in the present invention will contain 50-500,000 IU/mL EPO, 0.01mM to 5mM phosphate buffer, 0.01mM to 150mM NaCl, 5mM to 50mM L-arginine or L-histidine or salt thereof, 0.001% to 0.1% (w/v) polysorbate 80, and 0.5mM to 50mM L-methionine, pH 5.0-7.0; and more preferably, 750-48,000 IU/mL EPO, 2mM phosphate buffer, 110mM NaCl, 43.1mM L-arginine HCl, 0.006 % (w/v) polysorbate 80, and 0.5, 1, 2, 3 or 5mM L-methionine, pH 6.0; or 2mM phosphate buffer, 142mM NaCl, 9.54mM L-histidine HCl, 0.006% (w/v) polysorbate 80, and 0.5, 1, 2, 3 or 5mM L-methionine, pH 6.0.

Also contemplated for use in inhibiting oxidation of methionine is nitrogen overlay. Nitrogen overlay can be introduced to the headspace of a vial or prefilled syringe by purging nitrogen during the filling process.

### Example 1

This example describes the preparation of EPO and NESP HSA containing and HSA-free single use and multi-dose formulations. The EPO and NESP protein preparations were prepared as described in the Materials and Methods section below.

NESP and/or EPO HSA-containing formulations were then prepared by adding 0.1-1% albumin, the appropriate buffering agents (e.g., sodium phosphate), and a tonicity modifier (e.g., sodium chloride) to the protein preparation to obtain formulations having the desired concentrations of protein and excipients. NESP and/or EPO HSA-free formulations were prepared by replacing the albumin with other recombinant proteins or pharmaceutically acceptable surfactants (e.g. polysorbate 20 or 80). Multi-dose formulations were prepared by introducing preservative(s) (e.g. benzyl alcohol) to the HSA-containing or HSA-free formulations.

### Example 2

This example describes experiments wherein the effect of free L-methionine on the aggregation (introduced by light) of NESP was evaluated. Although the underlying mechanism is not clear, under extreme conditions, light introduces significant aggregation to the NESP formulations. NESP single use, HSA-free formulations prepared as described in Example 1 were used in the experiment.

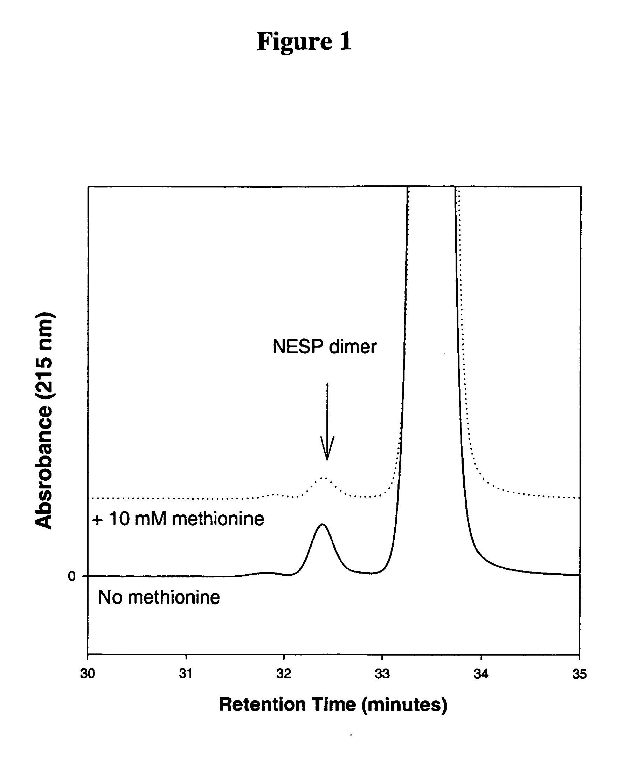

The glass vials containing the protein were placed into a UV light box and were incubated overnight (16 hours) with continuous UV light exposure. The samples were analyzed with SEC-HPLC. As depicted in Figure 1, addition of 10mM free methionine significantly decreased the rate of aggregation.

### Example 3

This example describes experiments wherein the effect of free L-methionine on the aggregation of NESP in the presence of benzyl alcohol was evaluated. Although the underlying mechanism is not clear, benzyl alcohol introduces very minor aggregation to the NESP formulations even during storage at 2-8ºC. NESP multi-dose, HSA-free formulations prepared as described in Example 1 were used in the experiment.

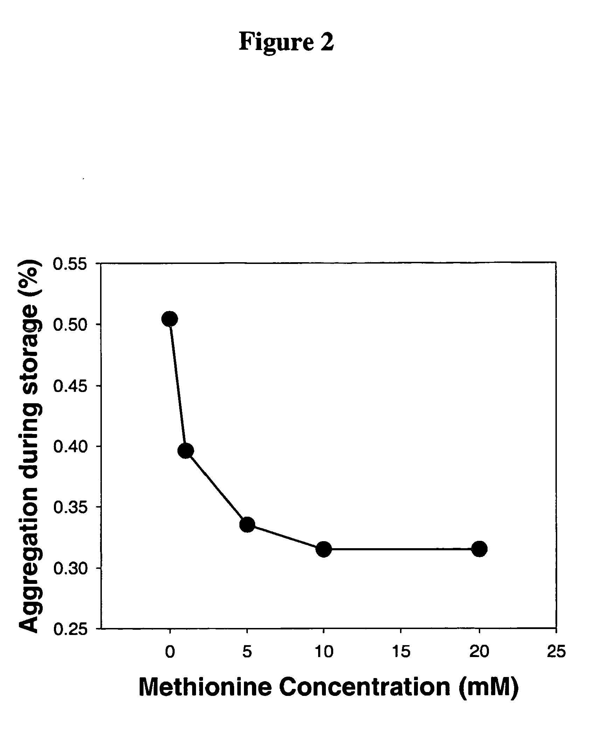

Multi-dose formulations containing 1% benzyl alcohol were incubated for 13 months at 2-8ºC and analyzed with SEC-HPLC method. As depicted in Figure 2, addition of 1mM-20mM free methionine significantly decreased the rate of aggregation.

### Example 4

This example describes experiments wherein various additives and treatments were tested for their ability to inhibit methionine oxidation in the NESP HSA-free single use formulations. NESP HSA-free single use formulations prepared as described in Example 1 were used in the experiments.

First, the protective effect of various anti-oxidants on NESP was examined by hydrogen peroxide spiking experiment (described in the Materials and Methods section below). Free amino acids L-lysine, glycine and L-methionine were tested and the % oxidation was determined by tryptic mapping as described in the Materials and Methods section below. It was demonstrated convincingly that free L-methionine prevents the oxidation of the Met-54 residue of NESP in the presence of excess hydrogen peroxide (see Table 1).

**Table 1**

| Anti-Oxidant | NESP Met-54 Oxidation (%) |
|---|---|
| Glycine | 100 |
| Lysine | 100 |
| Methionine | 37.3 |
| Glycine + Lysine | 100 |
| Glycine + Methionine | 35.3 |
| Lysine + Glycine + Methionine | 32.9 |

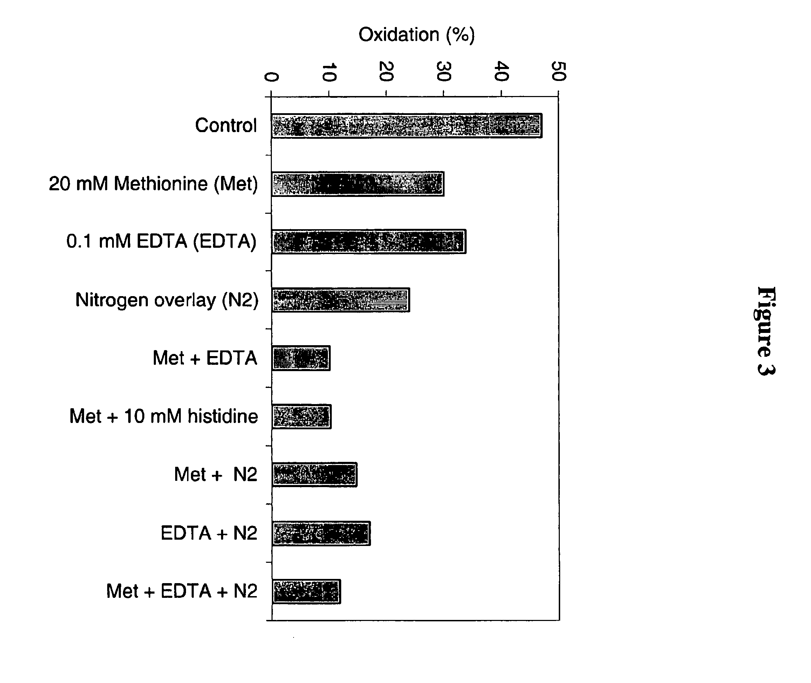

Next, the protective effect of various additives and treatments on NESP was examined. A NESP HSA-free formulation was used as a control. Additives tested were 20mM L-Methionine, 10mM histidine and 0.1mM EDTA. Nitrogen overlay in the head space was also evaluated. It was determined that free L-Methionine, EDTA, histidine, and/or nitrogen overlay can effectively inhibit the oxidation of Met-54 residue of NESP HSA-free formulations against various oxidative agents such as peroxide, superoxide ions (see Figure 3). The combination of free L-Methionine with either EDTA or histidine was more effective in inhibiting the oxidation than individual additives (see Figure 3). The combination of free L-Methionine and nitrogen overlay in the head space was also more effective in individual treatment (see Figure 3).

### Example 5

This example describes experiments wherein various additives and treatments were tested for their ability to inhibit methionine oxidation in EPO and/or NESP HSA-free multi-dose formulations. EPO and/or NESP HSA-free multi-dose formulations prepared as described in Example 1 were used in the experiments.

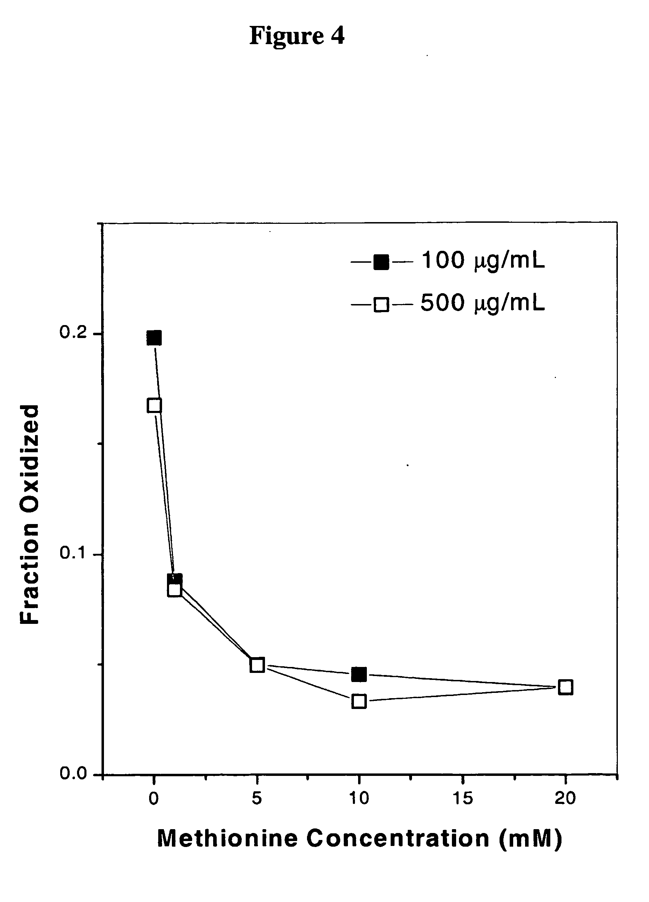

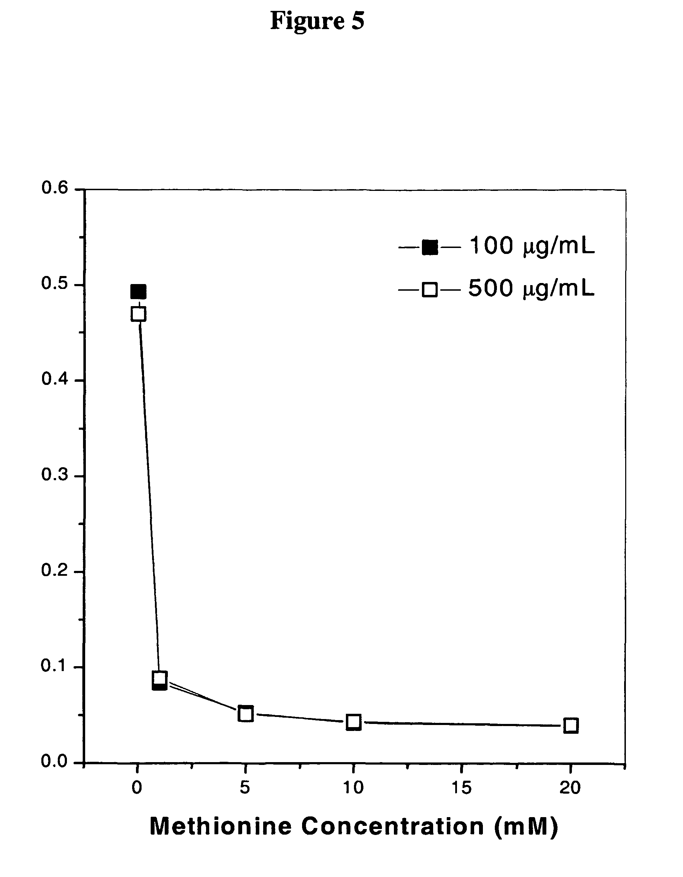

First, the protective effect of various concentrations of free L-Methionine on NESP HSA-free multi-dose formulations was examined by hydrogen peroxide spiking experiments as described in Example 2. The formulations contained 1% benzyl alcohol and free methionine concentrations ranging form 0-20mM were tested. Samples were incubated for 56 days at either 4°C or 29°C. The addition of free L-Methionine was found to be effective in inhibiting the oxidation induced by benzyl alcohol impurity in the multi-dose formulation (see Figures 4 and 5).

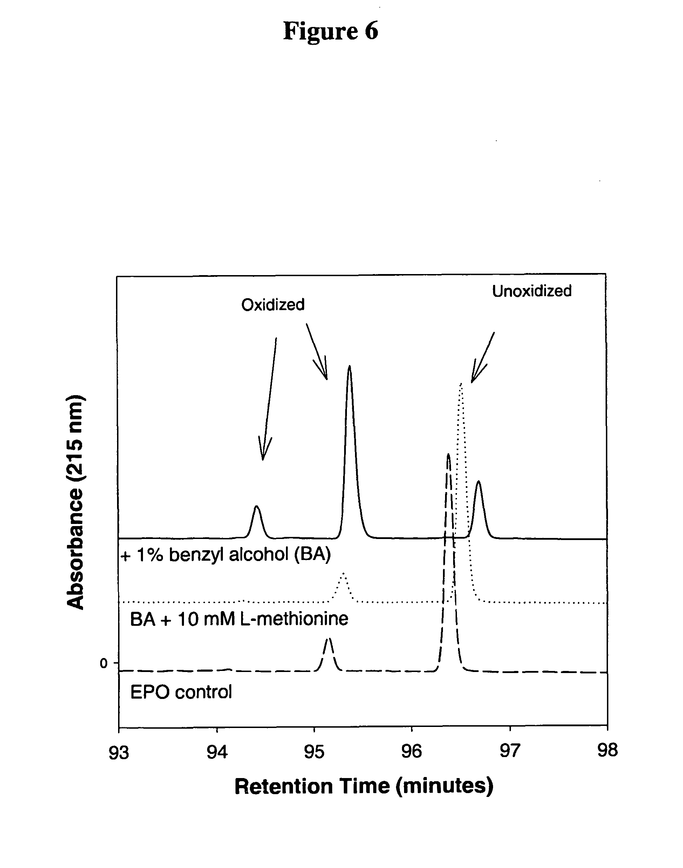

Next, the effect of methionine on HSA-free EPO formulations ± benzyl alcohol was evaluated. Figure 6 compares the tryptic maps of EPO in solutions with and without benzyl alcohol, and it is clear that the addition of this particular lot of benzyl alcohol can lead to nearly complete oxidation of EPO in solution at pH 7.0. However, the addition of free L-Methionine can completely prevent the oxidation of EPO in a solution containing the same benzyl alcohol.

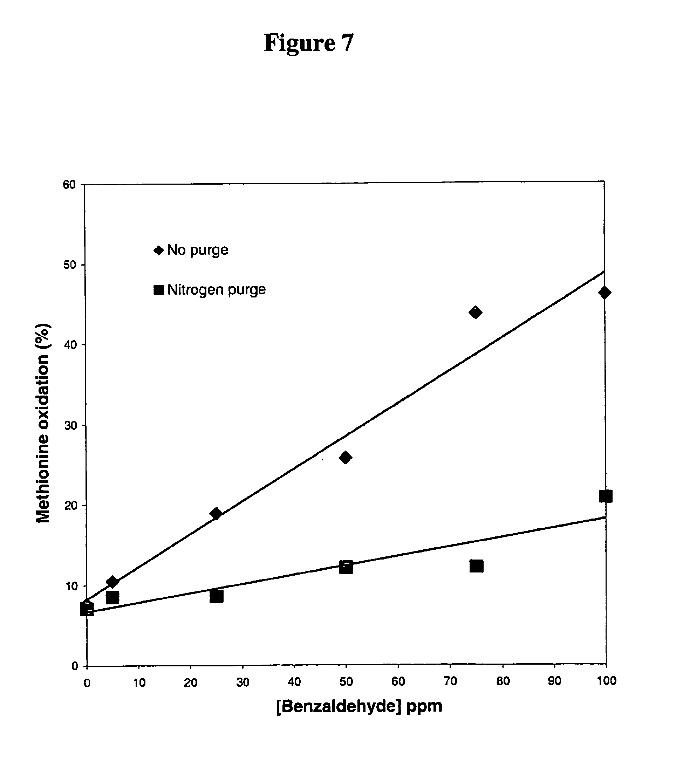

In addition, it was determined that purging the buffer solution with nitrogen could also significantly reduce the rate of Met-54 oxidation of NESP by benzaldehyde (see Figure 7). This indicates that free L-Methionine can inhibit the oxidative effect of dissolved molecular oxygen on Met-54 of NESP.

### Example 6

This example describes experiments wherein the effect of methionine 54 oxidation on the biological activity of NESP was evaluated. First, NESP formulations were oxidized with 0.01% hydrogen peroxide for different duration such that NESP samples containing different amounts of oxidized methionine 54 residue could be obtained. It was determined that the oxidation of methionine 54 does not adversely affect biological activity of NESP or EPO (see Table 2).

**Table 2**

| | Activity (%) | |
|---|---|---|
| Oxidation (%) | *In vitro* | *in vivo* |
| Control | 121 | 121 |
| 15 | 92 | 133 |
| 39 | 95 | 125 |
| 57 | 90 | 109 |
| 76 | 102 | 100 |
| 100 | 95 | 106 |

Next, a sufficient concentration of hydrogen peroxide was added and the samples incubated for several days such that all the methionine 54 residue in the NESP solution are oxidized even in the presence of added free L-methionine. It was determined that under extreme oxidative stress, NESP loses biological activity, in that samples that did not contain free methionine lost significant biological activity (see Table 3).

**Table 3**

| | Methionine | |
|---|---|---|
| Sample | Oxidation (%) | Activity (%) |
| 0mM Met, 0.25% H₂O₂, 6 days | 100 | 37 |
| 5mM Met, 0.25% H₂O₂, 6 days | 100 | 85 |
| 10mM Met, 0.25% H₂O₂, 6 days | 100 | 91 |
| 20mM Met, 0.25% H₂O₂, 6 days | 100 | 85 |
| 40mM Met, 0.25% H₂O₂, 6 days | 100 | 77 |

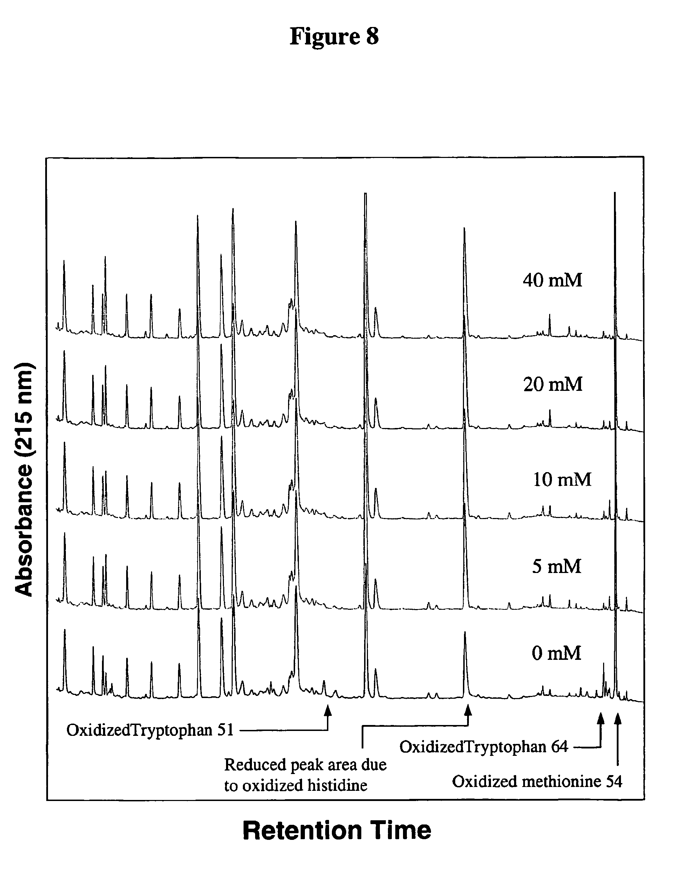

The inactivation of NESP was ascribed to the oxidation of other residues than methionine. Tryptophan, cysteine, and histidine were identified as additional oxidation sites (see Figure 8). Addition of free methionine prevents the oxidative inactivation of NESP by protecting these critical amino acids from oxidation (Table 3).

### Materials and Methods

The EPO used in the present invention may be prepared according to
U.S. Patent No. 4,703,008 (Lin).

The NESP used in the present invention may be prepared according to the
PCT Publication No. 95/05465.

Tryptic mapping of NESP or EPO was carried out by digesting the proteins with commercially available trypsin followed by separation of peptides with reversed-phase HPLC. A typical experiment would be carried out as follows: an aliquot of 20 µL trypsin digestion buffer, containing 20mM Methionine, 500mM Tris (Base), and 5M Urea at pH 8.2, will be added to 180 µL of sample followed by the addition of 4 µL of 1 mg/mL trypsin solution. After 18 hours of digestion at room temperature, the digested samples were analyzed by reversed-phase HPLC using a Phenomenex Jupiter C18 (250 x 4.6, 300 A) column.

Hydrogen peroxide spiking experiments were carried out by adding small aliquots of hydrogen peroxide to the sample to be tested. After incubation for a predetermined time at room temperature, the reaction was stopped by quenching free peroxide with the addition of 100mM excess free L-methionine.

The present invention has been described in terms of particular embodiments found or proposed to comprise preferred modes for the practice of the invention.

## Claims

1. A pharmaceutical formulation comprising a novel erythropoiesis stimulating protein and further comprising methionine.

2. A formulation according to claim 1 wherein said formulation does not contain human serum albumin

3. A formulation according to claim 1 or claim 2 wherein said methionine is present in a concentration of about 0.5 to 50mM.

4. A formulation according to claim 1 or claim 2 further comprising a pH buffering agent which provides a pH range of about 5 to about 7.

5. A formulation according to claim 4 further comprising a stabilizing amount of a sorbitan mono-9-octadecenoate poly(oxy-1,2-ethanediyl) derivative which is present in a concentration of about 0.001% to 0.1 % (w/v).

6. A formulation according to claim 1 or claim 2 wherein said protein is novel erythropoiesis stimulating protein (NESP) or a chemically modified form thereof.

7. A formulation according to claim 6 wherein said NESP has an amino acid sequence as depicted in SEQ ID NO: 2.

8. A formulation according to claim 7 further comprising a pH buffering agent which provides a pH range of about 5 to about 7.

9. A formulation according to claim 8 further comprising a stabilizing amount of a sorbitan mono-9-octadecenoate poly(oxy-1,2-ethanediyl) derivative which is present in a concentration of about 0.001% to 0.1% (w/v).

10. A pharmaceutical multi-dose formulation comprising a novel erythropoiesis stimulating protein, a preservative, and methionine, wherein said formulation demonstrates improved stability.

11. A formulation according to claim 10 wherein said formulation does not contain human serum albumin.

12. A formulation according to claim 10 or claim 11 wherein said methionine is present in a concentration of about 0.5mM to 50 mM.

13. A formulation according to claim 10 or claim 11 wherein said preservative is benzyl alcohol which is present in a concentration of about 0% to 2% (w/v).

14. A formulation according to claim 13 further comprising a pH buffering agent which provides a pH range of about 7 to about 7.

15. A formulation according to claim 14 further comprising a stabilizing amount of a sorbitan mono-9-octadecenoate poly(oxy-1,2-ethanediyl) derivative which is present in a concentration of about 0.001% to 0.1% (w/v).

16. A formulation according to claim 10 or claim 11 wherein said protein is novel erythropoiesis stimulating protein (NESP) or a chemically modified form thereof.

17. A formulation according to claim 16 wherein said NESP has an amino acid sequence as depicted in SEQ ID NO: 2.

18. A formulation according to claim 17 wherein said preservative is benzyl alcohol which is present in a concentration of about 0% to 2% (w/v).

19. A formulation according to claim 18 further comprising a pH buffering agent which provides a pH range of about 5 to about 7.

20. A formulation according to claim 19 further comprising a stabilizing amount of a sorbitan mono-9-octadecenoate poly(oxy-1,2-ethanediyl) derivative which is present in a concentration of about 0.001% to 0.1 % (w/v).

21. A method of stabilizing a pharmaceutical composition of a protein selected from erythropoietin (EPO) or novel erythropoiesis stimulating protein (NESP) against tryptophan, cysteine and/or histidine oxidation which comprises adding methionine to said composition in an amount sufficient to inhibit oxidation of an amino acid residue in the amino acid sequence of said EPO or NESP; wherein said amino acid residue is selected from the group consisting of tryptophan, cysteine, histidine and combinations thereof.

22. A method of claim 21 wherein said pharmaceutical composition does not contain human serum albumin.

23. A method of claim 21 wherein said protein is erythropoietin protein.

24. A method of claim 21 wherein said protein is novel erythropoiesis stimulating protein.

25. A pharmaceutical formulation comprising a protein selected from erythropoietin (EPO) or novel erythropoiesis stimulating protein (NESP) and methionine present in a concentration of about 0.5mM to 50mM, wherein said formulation demonstrates improved stability.

26. A formulation according to claim 25 wherein said protein is erythropoietin protein.

27. A formulation according to claim 25 wherein said protein is novel erythropoiesis stimulating protein.

## Patentansprüche

1. Pharmazeutische Formulierung, umfassend ein neues Erythropoesestimulierendes Protein und außerdem Methionin umfassend.

2. Formulierung gemäß Anspruch 1, wobei diese Formulierung kein humanes Serumalbumin enthält.

3. Formulierung gemäß Anspruch 1 oder Anspruch 2, wobei das Methionin in einer Konzentration von etwa 0,5 bis 50 mM vorhanden ist.

4. Formulierung gemäß Anspruch 1 oder Anspruch 2, außerdem umfassend ein pH-Puffermittel, welches einen pH-Bereich von etwa 5 bis etwa 7 bereitstellt.

5. Formulierung gemäß Anspruch 4, außerdem umfassend eine stabilisierende Menge eines Sorbitan-mono-9-octadecenoat-poly(oxy-1,2-ethandiyl)-Derivats, welches in einer Konzentration von etwa 0,001% bis 0,1% (w/v) vorhanden ist.

6. Formulierung gemäß Anspruch 1 oder Anspruch 2, wobei das Protein neues Erythropoese-stimulierendes Protein (NESP) oder eine chemisch modifizierte Form davon ist.

7. Formulierung gemäß Anspruch 6, wobei das NESP eine Aminosäuresequenz aufweist, wie dargestellt in SEQ ID NO: 2.

8. Formulierung gemäß Anspruch 7, außerdem umfassend ein pH-Puffermittel, welches einen pH-Bereich von etwa 5 bis etwa 7 bereitstellt.

9. Formulierung gemäß Anspruch 8, außerdem umfassend eine stabilisierende Menge eines Sorbitan-mono-9-octadecenoat-poly(oxy-1,2-ethandiyl)-Derivats, welches in einer Konzentration von etwa 0,001% bis 0,1% (w/v) vorhanden ist.

10. Pharmazeutische Vielfachdosen-Formulierung, umfassend ein neues Erythropoese-stimulierendes Protein, ein Konservierungsmittel und Methionin, wobei diese Formulierung verbesserte Stabilität aufweist.

11. Formulierung gemäß Anspruch 10, wobei diese Formulierung kein humanes Serumalbumin enthält.

12. Formulierung gemäß Anspruch 10 oder Anspruch 11, wobei das Methionin in einer Konzentration von etwa 0,5 mM bis 50 mM vorhanden ist.

13. Formulierung gemäß Anspruch 10 oder Anspruch 11, wobei das Konservierungsmittel Benzylalkohol ist, welches in einer Konzentration von etwa 0% bis 2% (w/v) vorhanden ist.

14. Formulierung gemäß Anspruch 13, außerdem umfassend ein pH-Puffermittel, welches einen pH-Bereich von etwa 5 bis etwa 7 bereitstellt.

15. Formulierung gemäß Anspruch 14, außerdem umfassend eine stabilisierende Menge eines Sorbitan-mono-9-octadecenoat-poly(oxy-1,2-ethandiyl)-Derivats, welches in einer Konzentration von etwa 0,001% bis 0,1% (w/v) vorhanden ist.

16. Formulierung gemäß Anspruch 10 oder Anspruch 11, wobei das Protein ein neues Erythropoese-stimulierendes Protein (NESP) oder eine chemisch modifizierte Form davon ist.

17. Formulierung gemäß Anspruch 16, wobei das NESP eine Aminosäuresequenz aufweist, wie dargestellt in SEQ ID NO: 2.

18. Formulierung gemäß Anspruch 17, wobei das Konservierungsmittel Benzylalkohol ist, welches in einer Konzentration von etwa 0% bis 2% (w/v) vorhanden ist.

19. Formulierung gemäß Anspruch 18, außerdem umfassend ein pH-Puffermittel, welches einen pH-Bereich von etwa 5 bis etwa 7 bereitstellt.

20. Formulierung gemäß Anspruch 19, außerdem umfassend eine stabilisierende Menge eines Sorbitan-mono-9-octadecenoat-poly(oxy-1,2-ethandiyl)-Derivats, welches in einer Konzentration von etwa 0,001 % bis 0,1% (w/v) vorhanden ist.

21. Verfahren zur Stabilisierung einer pharmazeutischen Zusammensetzung eines Proteins, ausgewählt aus Erythropoetin (EPO) oder einem neuen Erythropoesestimulierenden Protein (NESP) gegen Tryptophan-, Cystein- und/oder Histidin-Oxidation, welches das Zugeben von Methionin zu der Zusammensetzung in einer ausreichenden Menge umfasst, um die Oxidation eines Aminosäurerestes in der Aminosäuresequenz des EPO oder NESP zu hemmen; wobei der Aminosäurerest ausgewählt ist aus der Gruppe, bestehend aus Tryptophan, Cystein, Histidin und Kombinationen daraus.

22. Verfahren gemäß Anspruch 21, wobei die pharmazeutische Zusammensetzung kein humanes Serumalbumin enthält.

23. Verfahren gemäß Anspruch 21, wobei das Protein Erythropoetin-Protein ist.

24. Verfahren gemäß Anspruch 21, wobei das Protein neues Erythropoesestimulierendes Protein ist.

25. Pharmazeutische Zusammensetzung, umfassend ein Protein, das ausgewählt ist aus Erythropoetin (EPO) oder neuem Erythropoese-stimulierendem Protein (NESP), und Methionin, das in einer Konzentration von etwa 0,5 mM bis 50 mM vorliegt, wobei diese Formulierung verbesserte Stabilität aufweist.

26. Formulierung gemäß Anspruch 25, wobei das Protein Erythropoetin-Protein ist.

27. Verfahren gemäß Anspruch 25, wobei das Protein neues Erythropoesestimulierendes Protein ist.

## Revendications

1. Formulation pharmaceutique comprenant une nouvelle protéine stimulant l'érythropoïèse et comprenant en outre de la L-méthionine.

2. Formulation selon la revendication 1, ladite formulation ne contenant pas de sérum-albumine humaine.

3. Formulation selon la revendication 1 ou la revendication 2, dans laquelle ladite L-méthionine est présente dans une concentration d'environ 0,5 à 50 mM.

4. Formulation selon la revendication 1 ou la revendication 2, comprenant en outre un tampon de pH qui fournit une plage de pH d'environ 5 à environ 7.

5. Formulation selon la revendication 4, comprenant en outre une quantité stabilisante d'un dérivé de poly(oxy-1,2-éthanediyle) du mono-9-octadécénoate de sorbitan qui est présent dans une concentration d'environ 0,001% à 0,1% (m/v).

6. Formulation selon la revendication 1 ou la revendication 2, dans laquelle ladite protéine est une nouvelle protéine stimulant l'érythropoïèse (NESP) ou une de ses formes chimiquement modifiées.

7. Formulation selon la revendication 6, dans laquelle ladite NESP présente une séquence d'acides aminés telle qu'indiquée dans la SEQ ID NO : 2.

8. Formulation selon la revendication 7, comprenant en outre un tampon de pH qui fournit une plage de pH d'environ 5 à environ 7.

9. Formulation selon la revendication 8, comprenant en outre une quantité stabilisante d'un dérivé de poly(oxy-1,2-éthanediyle) du mono-9-octadécénoate de sorbitan qui est présent dans une concentration d'environ 0,001% à 0,1% (m/v).

10. Formulation multi-dose pharmaceutique comprenant une nouvelle protéine stimulant l'érythropoïèse, un conservateur et de la L-méthionine, ladite formulation faisant preuve d'une stabilité améliorée.

11. Formulation selon la revendication 10, ladite formulation ne contenant pas de sérum-albumine humaine.

12. Formulation selon la revendication 10 ou la revendication 11, dans laquelle ladite L-méthionine est présente dans une concentration d'environ 0,5 mM à 50 mM.

13. Formulation selon la revendication 10 ou la revendication 11, dans laquelle ledit conservateur est de l'alcool benzylique qui est présent dans une concentration d'environ 0% à 2% (m/v).

14. Formulation selon la revendication 13, comprenant en outre un tampon de pH qui fournit une gamme de pH d'environ 5 à environ 7.

15. Formulation selon la revendication 14, comprenant en outre une quantité stabilisante d'un dérivé de poly(oxy-1,2-éthanediyle) du mono-9-octadécénoate de sorbitan qui est présent dans une concentration d'environ 0,001% à 0,1% (m/v).

16. Formulation selon la revendication 10 ou la revendication 11, dans laquelle ladite protéine est une nouvelle protéine stimulant l'érythropoïèse (NESP) ou une de ses formes chimiquement modifiées.

17. Formulation selon la revendication 16, dans laquelle ladite NESP présente une séquence d'acides aminés telle qu'indiquée dans la SEQ ID NO : 2.

18. Formulation selon la revendication 17, dans laquelle ledit conservateur est de l'alcool benzylique qui est présent dans une concentration d'environ 0% à 2% (m/v).

19. Formulation selon la revendication 18, comprenant en outre un tampon de pH qui fournit une plage de pH d'environ 5 à environ 7.

20. Formulation selon la revendication 19, comprenant en outre une quantité stabilisante d'un dérivé de poly(oxy-1,2-éthanediyle) du mono-9-octadécénoate de sorbitan qui est présent dans une concentration d'environ 0,001% à 0,1% (m/v).

21. Procédé de stabilisation d'une composition pharmaceutique d'une protéine choisie parmi une érythropoïétine (EPO) ou une nouvelle protéine stimulant l'érythropoïèse (NESP) contre l'oxydation du tryptophane, de la cystéine et/ou de l'histidine qui comprend l'ajout de L-méthionine à ladite composition dans une quantité suffisante pour inhiber l'oxydation d'un résidu d'acide aminé dans la séquence d'acides aminés de ladite EPO ou NESP ; dans lequel ledit résidu d'acide aminé est choisi dans le groupe constitué de tryptophane, de cystéine, d'histidine et de combinaisons de ceux-ci.

22. Procédé selon la revendication 21, dans lequel ladite composition pharmaceutique ne contient pas de sérum-albumine humaine.

23. Procédé selon la revendication 21, dans lequel ladite protéine est une protéine érythropoïétine.

24. Procédé selon la revendication 21, dans lequel ladite protéine est une nouvelle protéine stimulant l'érythropoïèse.

25. Formulation pharmaceutique comprenant une protéine choisie parmi une érythropoïétine (EPO) ou une nouvelle protéine stimulant l'érythropoïèse (NESP) et une L-méthionine présente dans une concentration d'environ 0,5 mM à 50 mM, ladite formulation faisant preuve d'une stabilité améliorée.

26. Formulation selon la revendication 25, dans laquelle ladite protéine est une protéine érythropoïétine.

27. Formulation selon la revendication 25, dans laquelle ladite protéine est une protéine stimulant l'érythropoïèse.


### SEQUENCE LISTING

<110> Li, Tiansheng
   Chang, Byeong
   sloey, Christopher
<120> L-METHIONINE AS A STABILIZER FOR NESP/EPO IN HSA-FREE FORMULATIONS
<130> A-803
<140> TBD
   <141> 2001-08-30
<160> 2
<170> PatentIn version 3.0
<210> 1
   <211> 165
   <212> PRT
   <213> Homo sapiens
<400> 1
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Ala Glu His
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Pro Trp Glu Pro Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
               115                     120                     125
   Pro Leu Arg Thr Ile Thr Ala Asp Thr Phe Arg Lys Leu Phe Arg Val
       130                     135                     140
   Tyr Ser Asn Phe Leu Arg Gly Lys Leu Lys Leu Tyr Thr Gly Glu Ala
   145                 150                     155                 160
   Cys Arg Thr Gly Asp
                   165
<210> 2
   <211> 165
   <212> PRT
   <213> Homo sapiens
<400> 2
   Ala Pro Pro Arg Leu Ile Cys Asp Ser Arg Val Leu Glu Arg Tyr Leu
   1               5                   10                  15
   Leu Glu Ala Lys Glu Ala Glu Asn Ile Thr Thr Gly Cys Asn Glu Thr
               20                  25                  30
   Cys Ser Leu Asn Glu Asn Ile Thr Val Pro Asp Thr Lys Val Asn Phe
           35                  40                  45
   Tyr Ala Trp Lys Arg Met Glu Val Gly Gln Gln Ala Val Glu Val Trp
       50                  55                  60
   Gln Gly Leu Ala Leu Leu Ser Glu Ala Val Leu Arg Gly Gln Ala Leu
   65                  70                  75                  80
   Leu Val Asn Ser Ser Gln Val Asn Glu Thr Leu Gln Leu His Val Asp
                   85                  90                  95
   Lys Ala Val Ser Gly Leu Arg Ser Leu Thr Thr Leu Leu Arg Ala Leu
               100                 105                 110
   Gly Ala Gln Lys Glu Ala Ile Ser Pro Pro Asp Ala Ala Ser Ala Ala
           115                 120                 125
   Pro Leu Arg Thr Ile Thr Ala Asp Thr Phe Arg Lys Leu Phe Arg Val
       130                 135                 140
   Tyr Ser Asn Phe Leu Arg Gly Lys Leu Lys Leu Tyr Thr Gly Glu Ala
   145                 150                 155                 160
   Cys Arg Thr Gly Asp
                   165